(19) Europäisches Patentamt European Patent Office Office européen des brevets

(11) **EP 3 648 110 A1**

(12) **EUROPEAN PATENT APPLICATION**

(43) Date of publication:
06.05.2020 Bulletin 2020/19

(51) Int Cl.:
*G16B 20/20* (2019.01)    *G16H 50/20* (2018.01)

(21) Application number: 18306444.3

(22) Date of filing: 02.11.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Immune Compass Ltd.**
**London E1 6BT (GB)**

(72) Inventors:
• **MORA SANCHEZ, Aldo**
**LONDON, E1 6BT (GB)**
• **AGUILAR SALVADOR, Daniel Isui**
**75014 PARIS (FR)**

(74) Representative: **Regimbeau**
**20, rue de Chazelles**
**75847 Paris Cedex 17 (FR)**

(54) **SYSTEM FOR SCREENING FOR A DISORDER LINKED TO THE IMMUNE SYSTEM**

(57) The present invention relates to a system for calculating a value representative of a probability for a disorder linked to the immune system to affect a developing organism, configured for carrying the steps of calculating the values of a set of parameters, each parameter being a numerical representation of at least a gene allele, the values of the parameters being calculated from at least two data sets, said data sets being representative of gene alleles of different progenitors of the developing organism, and calculating the value representative of a probability for a disorder linked to the immune system to affect a developing organism from the values of the parameters calculated by means of a classifier built by automatic learning from a reference data set representative of genes alleles of the reference progenitors.

Figure 2

**Description**

**TECHNICAL FIELD**

The present invention relates to a system configured for screening for a disorder linked to the immune system, and more particularly for screening *a priori* an obstetric complication such as a miscarriage, a recurrent spontaneous abortion, an implantation failure, a stillbirth and/or disorders of the offspring of progenitors, said disorders being linked to the immune system.

**BACKGROUND OF THE INVENTION**

Naturally occurring couples are not randomly matched at the immune system level. For given maternal and paternal alleles of immune system related genes of a developing organism, particular combinations occur more often than others. Viable combinations occur more often, as non-viable combinations might lead to an unsuccessful pregnancy. Elsewhere, there has been selective pressure to increase diversity on the HLA gene complex. The way such combinations occur is known to affect pregnancy and health of the offspring.

Infectious agents are proposed as the strongest selective force shaping human evolution. 1,400 parasitic species infect humans, and in 2010, parasites were responsible for nearly 64% of global deaths in children younger than 5 years, as evaluated by Markov *et al.* [1].

HLA is the most polymorphic region of the human genome, potentially because slowly evolving vertebrates defend against rapidly evolving microparasites. Since each HLA type is potentially able to act against different sets of pathogens, heterozygous individuals can be favored by natural selection as evaluated by Markov *et al.* [1].

Heterozygosity is further promoted through reproductive mechanisms. Studies in animals and humans of Winternitz *et al.* [2] suggest that the mating behavior favors HLA gene diversity of a couple of progenitors, or of the progenitor himself.

In addition, couples more dissimilar in their HLA alleles have been shown to be more fertile (Ober, [3]).

On the contrary, embryos can be considered as semiallografts in the mother's fetus and face the risk of immune rejection at different stages. Therefore, HLA diversity can be limited by developmental factors.

*Embryo implantation likelihood*

Nowak *et al.* [4] describe how natural killer (NK) cells constitute the dominant cell population in the endometrium and are in close contact with the allogeneic extravillous trophoblast cells in early pregnancy decidua. These cells express killer immunoglobulin-like receptors (KIRs), which interact with HLA-C and HLA-G proteins on trophoblasts, either by stimulating or inhibiting NK cells, and thus allowing the low cytotoxicity required for maintenance of the embryo and fetus during pregnancy.

Hiby *et al.* [5] found evidence of the effect on reproductive success of complex interactions between maternal activating KIR and fetal (paternally derived) HLA-C types. In particular they discuss combinations favourable for placentation. In addition, different associations between maternal HLA and implantation failures have been described. For instance, Lashley *et al.* [6] describe how HLA-C2 allotype and the HLA-G allele with a 14 bp insertion correlate with implantation failures.

*Probability of miscarriage*

Persson *et al.* [7] describe the effects of the expression of HLA class lb proteins at the feto-maternal interface in the placenta. The evidence suggests a role of HLA class lb (especially HLA-G, due to immune-modulatory functions) throughout the reproductive cycle from conception to the birth weight of the child. In particular, the expression of HLA class lb proteins seems to influence the risk of recurrent spontaneous abortions, and the success in IVF treatments.

In addition, recent data have revealed that HLA-F interacts with both activating and inhibitory receptors on NK cells. As NK cells are very abundant in the uterus, in the endometrium, and at the feto-maternal contact zone during pregnancy, these interactions may also show to be of importance in human reproduction and might be involved in pathology behind infertility and preeclampsia.

Meuleman *et al.* [8] describe an increased risk of recurrent miscarriage in mothers carrying an HLA-DRB1*4 allele, a HLA-DRB1*15 allele, or a HLA-E*01:01 allele, and a decreased risk with HLA-DRB1*13 or HLA-DRB1*14 alleles. HLA-B sharing and HLA-DR sharing among parents were both associated with the occurrence of recurrent miscarriage.

Fan *et al.* [9] describe a significant association between HLA-G 14-bp polymorphism and patients with three or more miscarriages.

Other publications have found associations between recurrent miscarriage and HLA allele features, among which the frequency of heterozygous HLA-G*0105N null allele, HLA-E 0101/0103 heterozygous genotype, HLA-DQ2/DQ8 haplotype positivity, and the presence of HLA-DQA1*0102 and HLA-DQB1*0201 alleles.

The effect of HLA interactions is also known to be associated with pregnancy outcomes. For instance, interactions between HLA-G and uterine NK cells or CD8+ T cells or KIR and HLA genes is known.

*Probability of conceiving a healthy kid*

The weight of a human at birth is known to be an important predictor of both chances of survival of the baby, and of obstetric complications. Medium weights are associated with lower risks. Certain combinations of maternal/fetal immune system genes have been correlated with birth weight, for example an inhibitory maternal KIR AA genotype with a paternally derived fetal HLA-C2 ligand.

In addition, Emmery *et al.* [10] describe that an highest mean birth weight is associated to the HLA-G 14 bp insertion deletion/deletion gene polymorphism in heterozygous mothers. Conversely, lower weights are associated with 14 bp insertion/deletion.

MHC class I and II genes are also known to be associated with sexual selection in human and non-human primates. A significant preference for MHC diverse mates in humans has been demonstrated. This sexual selection mediated by the MHC is expected to maximize health.

Regarding general health, heterozygosity linked to the HLA-DRB1 is known to correlate with a decrease in symptoms of common infectious diseases.

However, the relevance of the correlation between immune-system related genes and pregnancy outcomes is still under question. The balance needed to optimize immune diversity while maintaining fetal tolerance is not yet known.

Statistical analysis of the prior art can establish plausible links between, for instance, treatments and outcomes, or genotypes and phenotypes, but do not provide a method for screening a possible disorder linked to the immune system at a personalized level.

The methods disclosed in the prior art do not allow for screening the risk of a particular obstetric complication, extremely relevant for example in the context of gamete donation.

## SUMMARY OF THE INVENTION

A system for calculating a value representative of a probability for a given disorder linked to the immune system to affect a developing organism issued from at least two progenitors, one of the at least two progenitors being an organism to be pregnant with the developing organism, has been developed to respond at least partially to the above-mentioned issues of the prior art.

The system comprises a processor unit and a memory unit, the processor unit being configured for carrying steps of:

  a) calculating values of a set of parameters, each parameter being a numerical representation of at least a gene allele, the values of the parameters being calculated from at least two data sets, said data sets being representative of:

  - gene alleles of the progenitors, one of the progenitors being the organism to be pregnant with the developing organism, or
  - gene alleles of the organism to be pregnant and gene alleles of the developing organism,

  , and said data sets being stored in the memory unit,
  b) calculating the value representative of a probability for the disorder linked to the immune system to affect the developing organism from the values of the set of parameters calculated at step a) by means of a classifier built by automatic learning from a reference data set representative of genes alleles of reference progenitors, the disorder linked to the immune system having affected only a portion of developing organisms issued from said reference progenitors.

As a prediction model, the classifier, is calculated from a reference data set representative of genes alleles of reference progenitors by automatic learning, it is possible to screen *a priori* the probability of a developing organism issued from progenitors to have a disorder linked to the immune system.

In further optional aspects of the invention:

  - the gene allele(s) are related to the immune system,
  - the disorder linked to the immune system is a disorder occurring during a pregnancy of one of the progenitors with the developing organism, said progenitors being selected from a couple of individuals and a couple of individuals and an egg donor,
- the system is configured for carrying the steps of acquiring the data sets prior step a),
- the developing organism is selected from an embryo and a fetus,
- the disorder linked to the immune system is an obstetrical complication selected from a miscarriage, a recurrent spontaneous abortion, an implantation failure and a stillbirth,
- at least one of the parameters is a numerical representation of both a gene allele and of the affinities of peptides to said gene allele,
- at least one of the parameters is a vector of a matrix calculated by decomposing an interaction matrix, each value of the interaction matrix corresponding to the affinity between a gene allele and a peptide,
- at least one predefined parameter of the set of parameters is a distance between gene alleles at a given locus,
- at least one of the parameters of the set of parameters is a norm of a vector of distances between gene alleles at a given locus, and optionally the mean of distances between gene alleles at a given locus,
- the immune system related gene alleles are MHC gene alleles,
- the immune system related gene alleles are selected at least from an HLA gene allele and a KIR gene allele,
- the immune system related gene alleles are selected at least from HLA-A alleles, HLA-B alleles, HLA-C alleles, HLA-DRB1 alleles, HLA-DPB1 alleles, HLA-DQB1 alleles, HLA-E alleles, HLA-G alleles and KIR alleles,
- the immune system related gene alleles comprise a KIR gene haplotype and wherein the immune system related gene alleles are a a KIR AA haplotype, a KIR AB haplotype and a KIR BB haplotype, and wherein the values of the parameters of the parameters representing said gene alleles are equal,
- the progenitors comprise a potential progenitor selected from a set of potential progenitors, step b) is repeated for every potential progenitor of the set of potential progenitors, the processor unit being configured for further carrying a step c) of ranking the potential progenitors depending on the probability calculated at step b),
- the potential progenitor is selected from sperm donors and egg donors.

Another aspect of the present invention is a method for calculating a value representative of a probability for a disorder linked to the immune system to affect a developing organism issued from at least two progenitors, one of the at least two progenitors being an organism to be pregnant with the developing organism, comprising the steps of:

a) calculating values of a set of parameters, each parameter being a numerical representation of at least an immune system related gene allele, the values of the set of parameters being calculated from at least two data sets, said data sets being representative of:

- gene alleles of the progenitors, one of the progenitors being the organism to be pregnant with the developing organism, or
- gene alleles of the organism to be pregnant and gene alleles of the developing organism,

and said data sets being stored in the memory unit,
b) calculating the value representative of a probability for a disorder linked to the immune system to affect the developing organism from the values of the set of parameters calculated at step a) by means of a classifier built by automatic learning from a reference data set representative of immune system related genes alleles of reference progenitors, the disorder linked to the immune system having affected only a portion of developing organisms issued by said reference progenitors.

Another aspect of the present invention is a computer program product, comprising computer instructions for carrying out the method previously described.
Another aspect of the invention is a computer-readable storage medium having stored there in the computer program product previously described.
Another aspect of the invention is a system for building a classifier, said system comprising a processor unit and a memory unit, the memory unit comprising a reference data set, said reference data set comprising data representative of immune system related gene alleles of a plurality of reference progenitors, a disorder linked to the immune system having affected only a portion of developing organisms issued from the reference progenitors, said processor being configured for carrying the steps of:

e) calculating parameters from the reference data set, each parameter being a numerical representation of at least an immune system related gene allele,
f) selecting a subset of parameters among the parameters calculated at step e), the parameters of the selected subset being selected as being the most highly correlated to a disorder linked to the immune system,
g) training a classifier with the selected parameters of the selected subset.

## DEFINITIONS

The terms "developing organism" is preferably used herein to designate an animal at any development step, including an embryo, a fetus or a born or unborn child. Preferably, the animal is a human.

The term "progenitor" of a developing organism will be used herein to designate a father, a mother, an egg donor and/or a surrogate mother. The term "progenitor" can notably designate the future father, the future mother, the future egg donor and/or the future surrogate mother before the fecundation of the developing organism. The term "organism to be pregnant" will be used to designate the progenitor that is pregnant with the developing organism or that will be pregnant with the developing organism.

The terms "disorder linked to the immune system" is used herein to designate any disorder that is caused, at least partly, by the immune system, notably a miscarriage, a recurrent spontaneous abortion, an implantation failure, the health of the offspring of progenitors and/or a stillbirth. Obviously, when a disorder linked to the immune system affects a developing organism before the birth, the disorder is considered to also affect one of the progenitors, notably the mother or the surrogate mother. Therefore, a "disorder linked to the immune system affecting an unborn developing organism" comprises a disorder linked to the immune system affecting the mother or the surrogate mother of the developing organism.

The term "distance" is used herein to designate the dissimilarity between nucleic acid sequences, amino acid sequences, and/or more generally a set of alleles at the same loci within the same individual or at the same loci across different individuals. When the alleles are at the same loci within the same individual, they are at different physical chromosomes, the chromosomes having the same number. For example, the genetic distance is a measure of the genetic divergence between two different nucleic acid sequences: if the sequences are identical, the distance between the sequences is equal to zero.

## BRIEF DESCRIPTION OF THE DRAWINGS

The invention will be described by way of example, with reference to the accompanying drawings in which

- figure 1 diagrammatically shows a method for building a classifier according to an embodiment of the invention,
- figure 2 diagrammatically shows a method for building a classifier according to an embodiment of the invention by leave-one-out cross-validation,
- figure 3 diagrammatically shows the accuracy of a classifier depending on the number of selected parameters,
- figure 4 diagrammatically shows the sensitivity of a classifier of an embodiment of the invention depending on its specificity,
- figure 5 diagrammatically shows a method for calculating a value representative of a probability for a given disorder linked to the immune system to affect a developing organism issued from at least two progenitors, according to an embodiment of the invention.

## DETAILED DESCRIPTION OF PREFERRED ASPECTS OF THE INVENTION

*Reference data set*

A reference data set comprises data representative of gene alleles of a plurality of reference progenitors, and preferably of immune system related gene alleles of a plurality of reference progenitors. The data representative from gene alleles can be selected from nucleic acid sequences, amino acids sequences, a single nucleotide polymorphism (SNP) set, HLA protein names, HLA allele names, HLA protein groups, KIR names, KIR protein names, KIR allele names, KIR protein groups, and KIR haplotypes.

The disorder linked to the immune system can be an obstetrical complication selected from a miscarriage, a recurrent spontaneous abortion, an implantation failure and a stillbirth. Notably, the disorder linked to the immune system can be a miscarriage. For example, two hundred DNA samples from a previous study on spontaneous miscarriage can be used, for example from DNA samples from the study reported by Novak *et al.* [4], sequenced by DKMS Lab The reference progenitors (for example mother and fathers) were recruited from the Department of Surgical, Endoscopic and Oncologic Gynecology and Department of Gynecology and Gynecologic Oncology, Polish Mothers' Memorial Hospital-Research Institute, in Poland.

The reference data set comprises data representative of gene alleles of the progenitors of the developing organisms, one of the progenitors being the organism to be pregnant with the developing organism, notably of an embryo, affected by a disorder linked to the immune system, notably Recurrent Spontaneous Abortions (RSA). Those reference progenitors were free from chromosomal aberrations, uterine anomalies, hormonal disturbances, and infections with Toxoplasma, Chlamydia, Listeria, and Brucella, and were negative for the presence of autoantibodies.

The reference data set also comprises data representative of gene alleles of reference progenitors of developing organisms, one of the progenitors being the organism to be pregnant with the developing organism, notably babies, not affected by a disorder linked to the immune system (control group of progenitors). As an example, the control group was recruited from the 1st Department of Obstetrics and Gynecology, Medical University of Warsaw and from the Disctrict Hospital Strzelce Opolskie. This group consists in 219 healthy couples of progenitors with at least two healthy-born children and no history of miscarriage or endocrinological or immunological disorders: Experimental protocols were approved by the Local Ethics Committees (the agreement of Medical University of Wroclaw and Polish Mothers' Memorial Hospital-Research Institute in Łódź) and informed consent was obtained from all individual progenitors included in the study. Genomic DNA was isolated from venous blood using the Invisorb Spin Blood Midi Kit (Invitek, Berlin, Germany) following the manufacturer's instructions and stored until further use. HLA-A,B,C,DRB1,DQB1, DPB1,E and KIR high resolution genotyping was performed by DKMS Life Science Lab using in house Next Generation Sequencing.

The immune system related gene alleles can be selected at least from MHC gene alleles, notably from MHC class I and MHC class II gene alleles, notably from HLA gene allele and KIR allele, and preferably from HLA-A alleles, HLA-B alleles, HLA-C alleles, HLA-DRB1 alleles, HLA-DPB1 alleles, HLA-DQB1 alleles, HLA-E alleles, HLA-G alleles and KIR alleles. Therefore, the data set(s) can be representative of the main immune system related gene alleles, notably in humans,

*Parameters extraction*

Parameters representative of a disorder linked to the immune system and their values can be calculated from the data sets. The data sets can be representative of gene alleles of the progenitors of the developing organism(s), one of the progenitors being the organism to be pregnant with the developing organism, and preferably representative of immune system related genes alleles. Each parameter is a numerical representation of at least a gene allele, and preferably of an immune system related gene allele. Three types of parameters were generated: distance between alleles at each locus, parameters issued from protein continuous representations, and binary variables for the KIR of each parent. Therefore, it is possible to both reduce the cardinality of the information compared with considering the full information of the data set, without binarizing the data sets. This is made possible by calculating parameters having continuous values, *i.e.* belonging to all real numbers. Parameters corresponding to a distance between gene alleles and parameters being a representation of both a gene allele and of the affinities of peptides to said gene allele both share a common continuous representation of said alleles, *i.e.* continuous mapping of alleles.

The parameter can be a distance between gene alleles at a given locus, notably between immune system related gene alleles at a given locus. Preferably, the parameter can be a norm of the distances between gene alleles at a given locus, and preferably the mean of the distances between gene alleles at a given locus. For example, the distance can be calculated between different alleles of the same progenitor and/or between alleles of different progenitors. For example, for each locus, an average distance can be calculated considering four possible distances: each of two maternal copies against each of two paternal copies.

Preferably, the distance between two alleles at a given locus can be calculated by using a substitution matrix, for example a BLOSUM matrix, a PAM matrix or a PMBEC matrix.

The parameter can also be a numerical representation of both a gene allele and of affinities of peptides to said gene allele, and preferably of both an immune system related gene allele and of affinities of peptides to said immune system related gene allele. An interaction matrix can be used to calculate the parameter. An interaction matrix is used herein as a matrix describing the affinities between proteins and peptides. The interaction matrix can be built for example from the "Immune Epitope Database and Analysis Resource" which has a large collection of data regarding binding affinities between gene proteins and peptides. The proteins correspond for example to the rows of the matrix and the peptide to the columns. Each protein is related to a peptide in the interaction matrix by an affinity value, that was experimentally tested with said protein. The measure of the affinity can be for example the IC50 value or preferably 1 /log(IC50) value in order to scale the data. The use of the interaction matrix was inspired by the use of recommender systems in the technical field of movie ratings. An analogy can be made with recommender systems between, on one hand, the gene proteins and spectators, between, and on the other hand, the affinity and the rating of the spectators.

The interaction matrix A can be decomposed into two matrices Q and P. The decomposition can be made using the method of incremental SVD for example. The interaction matrix can have *n* lines corresponding to *n* gene proteins and m columns related to m peptides. It is possible to calculate by decomposition two matrices, a first matrix Q, having *n* lines and *r* columns, and a second matrix P, having *r* lines and m columns. *r* is called the rank of the first matrix and of the second matrix.

Any affinity $a_{i,j}$ can be written as in formula (1):

$$a_{i,j} = \mathbf{q}_i^{\mathsf{T}} \mathbf{p}_j \qquad\qquad (1)$$

wherein $\mathbf{q}_i$ is the vector corresponding to a given gene allele protein and $\mathbf{p}_j$ is a vector corresponding to a given peptide. In this embodiment of the invention, the elements of $\mathbf{q}_i$ can be chosen as parameters. $\mathbf{q}_i$ is a vector of the first matrix calculated by decomposition of the interaction matrix. Therefore, the parameter $\mathbf{q}_i$ comprises a set of r components which are representatives of the affinities of the $i^{th}$ gene protein in regard with the peptides used for building the interaction matrix. The values of the different components of $\mathbf{q}_i$ are continuous numbers belonging to real numbers. Therefore, the parameter is a representation of the interaction between the gene protein and the peptides.

The parameter can also be a binary variable, representative of the KIR haplotype, i.e. KIR alleles. Preferably, the value of the parameter is 1 if the KIR contained at least one of the KIR alleles: 2DL2, 2DL5, 2DS1, 2DS2, 2DS3, 2DS5, 3DS1 (this is called KIR B haplotype) and zero otherwise. Notably, individuals with at least one KIR B copy (haplotype AB and haplotype BB) are mapped to the same value of the parameter. Therefore, individuals homozygous for KIR A (KIR AA), known for lacking activating KIR genes, are mapped to the same parameter. Preferably, one parameter representative of KIR genes can be used per progenitor.

*Classifier building*

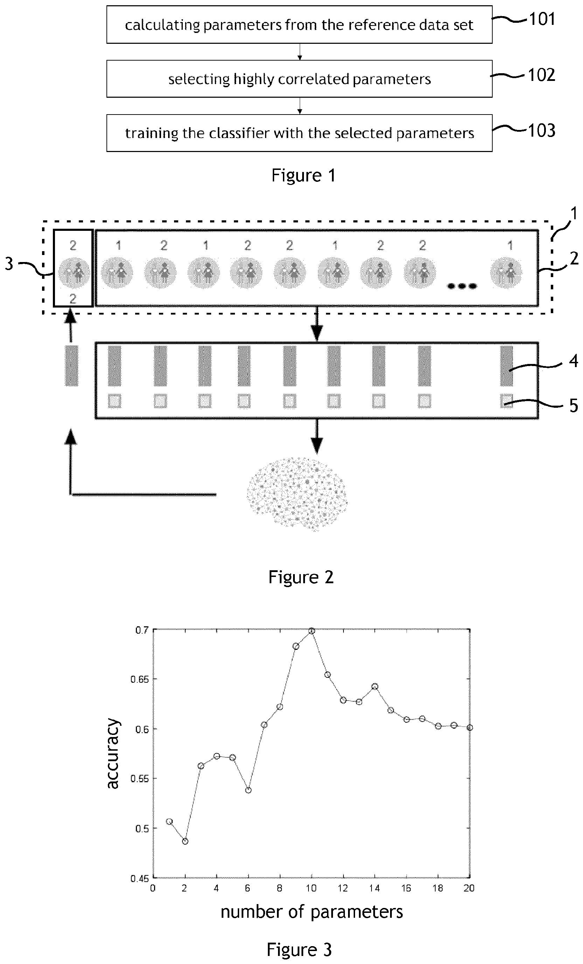

Referring to figure 1, a system for building a classifier comprises a processor unit and a memory unit. The memory unit comprises the reference data set. The processor of the system is configured for carrying the step 101 of calculating the parameters from the reference data set 1. A classifier is then built by automatic learning from the reference data set.

*Estimation of the performance relative to a data set*

Referring to figure 2, a cross-validation is performed to estimate the performance of the classifier from the reference data set 1. Preferably, a leave-one-out cross-validation is performed. The cross-validation can be used to predict the performance of the classifier built from the reference data set 1, and also for enhancing the performance of the classifier as well.

For estimating the performance of the classifier on the reference data set 1, the reference data set 1 is typically split into two parts, a training set 2 and a test set 3. The training set 2 is usually larger than the test set 3. The classifier is trained with the training set 2, and the performance is tested with the test set 3. The process is iterated for different partitions of the reference data set 1, and performances are averaged to provide a final estimation. In the leave-one-out version, the test set 3 consists of only one element, hence the process is iterated as many times as the number of elements of the reference data set 1. When done, the leave-one-out procedure has all the advantages of an independent data set of roughly the same size of the whole reference data set 1, with the only disadvantage that it is time consuming.

The processor is also configured for carrying a step 102 of selecting a portion of the parameters, the selected parameters 4 being the more highly correlated to a disorder linked to the immune system. For every cross-validation iteration, the parameter selection can be performed by Orthogonal Forward Regression (OFR) on the training data set 2. The parameters which best explain the output are chosen one by one, for example considering possible redundancy between them. If two parameters are correlated with the output, but both are highly correlated (linearly) to each other, only one of them is likely to be selected by OFR. The N more representative parameters are selected.

The system is configured for carrying the step 103 of training a classifier with the selected parameters. For example, a linear Support Vector Machine (SVM) is trained with the N selected parameters. Afterwards, the parameters calculated from the reference data set that was left out (corresponding to a disorder linked to the immune system or not) are calculated. The class (or group) to which said data belong to is calculated by said model. The latter generated a 1, or success event, if the class is correctly predicted, and a 0 otherwise. The process was iterated for each couple of progenitors, which gave rise to a mean accuracy that depended on N.

Referring to figure 3, N was varied from 1 to 20, and the number N that maximized the average classification accuracy on the whole reference data set was selected. In other words, N was chosen by cross-validation. The above-mentioned method allows to know, on average, the accuracy of the categorization process for new data, considering that the N parameters that best explain the output of the reference data set are selected to build the classifier used for calculating a value representative of a probability for a disorder linked to the immune system to affect a developing organism, from the data set representative of gene alleles of different progenitors to be screened.

*Assessment of statistical significance*

A label is attributed to each progenitor of the reference data set, notably to each group of progenitors giving rise to a developing organism. The label can be for example a binary value, indicating or not that the reference progenitor, or group of progenitors, is associated with a disorder linked to the immune system of the developing organism issued from the reference progenitor.

For estimating the statistical significance of the output of the classifier, the output corresponding to the null hypothesis is obtained by shuffling all the labels of all the reference data set. A permutation is performed to destroy any possible correlation between the parameters and the outputs. A classification accuracy is calculated. The shuffling process is repeated. Notably, the shuffling process is repeated 4000 times. The ratio between the number of times the accuracy of the model on the shuffled data is higher or equal than the one obtained in the unshuffled case, over the total number of iterations is calculated and corresponds to the p-value.

*Sensitivity optimization*

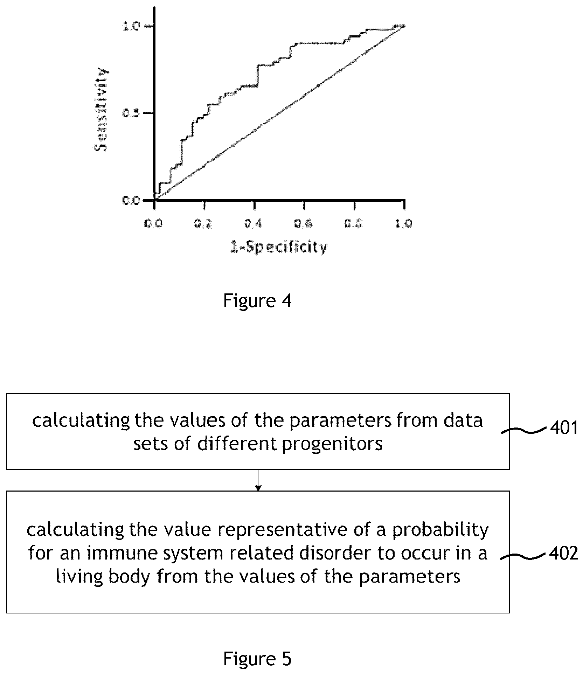

The output of the classifier can be a number between 0 and 1, this number being a probability that a disorder linked to the immune system to affect a developing organism issued from at least two progenitors. By choosing the class threshold above which a couple of reference progenitor is considered as belonging to one group or the other, the sensitivity or specificity can be increased at the expense of the other. Sensitivity can be preferably privileged over specificity. For example, when choosing a gamete donor, if a genetic combination is said to reduce the risk of an obstetric complication, we want to be sure that this is indeed the case. As there is usually a pool of donors, it is less important if by being strict, some potentially beneficial combinations are missed out. Referring to figure 3 and figure 4, when considering the 10 most important parameters obtained by the OFR, a sensitivity of 90% ($p = 0.03$) can be achieved if we accept a false positive rate of 0.57.

The sensitivity and specificity values diagrammatically shown in figure 4 are calculated for a case when the disorder linked to the immune system is an RSA. From the low p-value calculated, it is shown that immune related genes alleles are predictors of RSA. A natural consequence, regarding gamete donation, is that this evidence strongly suggests that immune related genetic matching works better than random matching. Random matching, at the genetic level, being matching whose criteria do not include genetic information, such as choosing a donor by height, education or ethnicity.

*Probability for a disorder linked to the immune system to occur*

Referring to figure 4, an aspect of the invention is a system for calculating a value representative of a probability for a disorder linked to the immune system to affect a developing organism issued from at least two progenitors. The system comprises a processor unit and a memory unit. The processor unit is configured for carrying the step 401 of calculating the values of a set of parameters, each parameter being a numerical representation of at least a gene allele.

The parameters are at least a distance between alleles at each locus, parameters issued from protein continuous representations, and binary variables for the KIR of each parent or any combination thereof. Preferentially, the parameters are chosen within distance between alleles at each locus, parameters issued from protein continuous representations. Therefore, the values of at least a portion of the parameters are continuous, and make it possible to both reduce the complexity of the information compared with considering the full information of the data sets, and reducing the complexity of the information. Preferably, the progenitors are selected at least from a couple of persons (for example the future mother and the future father of the future developing organism) and said couple of persons and an egg donor. The future father can be represented by a sperm sample from a sperm bank from instance.

Preferably, the parameters are the parameters selected in step 102.

The values of the parameters are calculated from at least two data sets. The values can be calculated from data sets representative of gene alleles of different progenitors of the developing organism, one of the progenitors being an organism to be pregnant with the developing organism. The progenitors can be the mother, the father and/or of an egg donor. The values can also be calculated from the data set representative of gene allele(s) of the organism to be pregnant with the developing organism and the data set representative of gene alleles of the developing organism, notably of the embryo. In the latest case, the data set representative of gene allele(s) of the developing organism comprises data representative of gene alleles of at least another progenitor than the mother, for example of the father. Preferably, said data set can be acquired, and stored in the memory. The data sets can for example be acquired by DNA sequencing of said alleles of each of the progenitors.

The processor is then configured for carrying the step 402 of calculating the value representative of a probability for a disorder linked to the immune system to affect a developing organism from the values of the parameters calculated at step 401, by means of the classifier, built by automatic learning in step 103.

The progenitors can notably comprise a potential progenitor selected from a set of potential progenitors. The set of potential progenitors can be a set of potential sperm donors, and/or a set of potential egg donors. Step b) can repeated for every potential progenitor of the set of potential progenitors. The processor unit can be configured for further carrying a step of ranking the potential progenitors depending on the probability calculated at step b). Therefore, the selection of a potential progenitor can be made so as to reduce the risk of a disorder linked to the immune system to affect the developing organism after a fecundation. The sperm donors can be ranked so as to minimize the probability. Notably, the mother can choose a sperm donor in a set of selected sperm donor, so as to avoid a disorder linked to the immune system, notably a miscarriage.

EXAMPLE

The extraction of DNA is performed from a couple of progenitors. The DNA is taken from blood.

Genotyping of the genes HLA-A, HLA-B, HLA-C, HLA-DRB1, HLA-DQB1, HLA-DPB1, and KIR of each of the progenitors is performed. The name of each of the alleles that each progenitor carries is provided. For instance, the typification results for one progenitor can be illustrated by the data set of table 1.

Table 1

| Gene | Allele |
|---|---|
| HLA-A | 25:01 |
| HLA-A | 29:02 |
| HLA-B | 18:01 |
| HLA-B | 44:03 |
| HLA-C | 12:03 |
| HLA-C | 16:01 |
| HLA-DRB1 | 07:01 |
| HLA-DRB1 | 15:01 |
| HLA-DQB1 | 02:01 |
| HLA-DQB1 | 06:02 |
| HLA-DPB1 | 04:01 |
| HLA-DPB1 | 23:01 |
| KIR | 11111101101011110 |

The first 12 lines of table 1 correspond to 6 HLA genes with two copies each, and the last line corresponds to the KIR gene. At least two data sets, corresponding to different progenitors have to be acquired.

The amino acid chain that constitutes the protein corresponding to a given allele is calculated. For instance, the above allele 25:01 corresponds to a protein whose chain of amino acids begins with the amino acids MAVM APRTLVLLLS.

The parameter corresponding to the distance between these two chains of amino acids is calculated by using the BLOSUM50 and the PMBEC substitution matrices.

The contribution of every position in which amino acids are changed can be considered, in order to provide a measurement of how different the two proteins are. The computer software Matlab built-in function computes distances using the substitution matrix. The mother has two copies of the HLA-A gene, so as the father. In order to provide a measure of how similar paternal and maternal alleles are at the HLA-A locus, the mean distance is calculated. Notably, four distances are calculated, the first copy of the mother with the first copy of the father, the first copy of the mother with the second copy of the father, the second copy of the mother with the first copy of the father, and the second copy of the mother with the second copy of the father. As the distances are calculated using two matrices, and there are 6 loci, there are 12 values of parameters measuring distances.

The six HLA genes can be represented by a single parameter each, the mean distance of the 6 preceding distances calculated for a given locus, whom value is a real number.

The parameters representative of the affinities of peptides to said immune system related gene allele are calculated from the data sets.

The affinity interaction matrix corresponding to the genes of table 1 are calculated. For each gene of table 1, the interaction matrix is decomposed into the product of a first matrix and a second matrix, using an incremental SVD method. The interaction matrix of size $n * m$ is decomposed into a first matrix of size $n * r$ and a second matrix of size $r * m$. $r$, the rank of the matrices, is chosen equal to three due to the relative low sample size. The chosen parameter is a vector corresponding for example to a line of the first matrix. The parameter is a vector comprising three numbers. This parameter is calculated for each of the HLA-C alleles of the progenitors.

Finally, the KIR of each member of the couple of progenitors was transformed into a binary variable. This variable was 1 if the KIR contained at least one of the KIR genes: 2DL2, 2DL5, 2DS1, 2DS2, 2DS3, 2DS5, 3DS1 (this is called KIR B haplotype) and zero otherwise. Therefore, KIR genes gave rise to two additional parameters, one from the mother, and one from the father.

**REFERENCES**

[1] Markov, P. V., & Pybus, O. G. (2015). Evolution and diversity of the human leukocyte antigen (HLA). Evolution, medicine, and public health, 2015(1), 1-1.

[2] Winternitz, J. C., & Abbate, J. L. (2015). Examining the evidence for major histocompatibility complex-dependent mate selection in humans and nonhuman primates. Research and Reports in Biology, 6, 73-88.

[3] Ober, C. (1999). Studies of HLA, sfertility and mate choice in a human isolate. Human Reproduction Update, 5(2), 103-107.

[4] Nowak, I., Wilczynska, K., Wilczynski, J. R., Malinowski, A., Radwan, P., Radwan, M., & Kusnierczyk, P. (2017). KIR, LILRB and their Ligands' Genes as Potential Biomarkers in Recurrent Implantation Failure. Archivum immunologiae et therapiae experimentalis, 65(5), 391-399.

[5] Hiby, S. E., Ashrafian-Bonab, M., Farrell, L., Single, R. M., Balloux, F., Carrington, M., & Moffett, A. (2010). Distribution of killer cell immunoglobulin-like receptors (KIR) and their HLA-C ligands in two Iranian populations. Immunogenetics, 62(2), 65-73.

[6] Lashley, L. E. E. L. O., van der Westerlaken, L. A. J., Haasnoot, G. W., Drabbels, J. J. M., Spruyt-Gerritse, M. J., Scherjon, S. A., & Claas, F. H. J. (2014). Maternal HLA-C2 and 14 bp insertion in HLA-G is associated with recurrent implantation failure after in vitro fertilization treatment. Tissue Antigens, 84(6), 536-544.

[7] Persson, G., Melsted, W. N., Nilsson, L. L., & Hviid, T. V. F. (2017). HLA class Ib in pregnancy and pregnancy-related disorders. Immunogenetics, 69(8-9), 581-595.

[8] Meuleman, T., Lashley, L. E., Dekkers, O. M., van Lith, J. M., Claas, F. H., & Bloemenkamp, K. W. (2015). HLA associations and HLA sharing in recurrent miscarriage: a systematic review and meta-analysis. Human immunology, 76(5), 362-373.

[9] Fan, W., Li, S., Huang, Z., & Chen, Q. (2014). Relationship between HLA-G polymorphism and susceptibility to recurrent miscarriage: a meta-analysis of non-family-based studies. Journal of assisted reproduction and genetics, 31(2), 173-184.

[10] Emmery, J., Christiansen, O. B., Nilsson, L. L., Dahl, M., Skovbo, P., Møller, A. M., ... & Hviid, T. V. F. (2017). Associations between fetal HLA-G genotype and birth weight and placental weight in a large cohort of pregnant women-Possible implications for HLA diversity. Journal of reproductive immunology, 120, 8-14.

**Claims**

1. A system for calculating a value representative of a probability for a given disorder linked to the immune system to affect a developing organism issued from at least two progenitors, one of the at least two progenitors being an organism to be pregnant with the developing organism, said system comprising a processor unit and a memory unit, the processor unit being configured for carrying steps of:

   a) calculating values of a set of parameters, each parameter being a numerical representation of at least a gene allele, the values of the parameters being calculated from at least two data sets, said data sets being representative of:

   - gene alleles of the progenitors, one of the progenitors being the organism to be pregnant with the developing organism, or
   - gene alleles of the organism to be pregnant and gene alleles of the developing organism,

   and said data sets being stored in the memory unit,
   b) calculating the value representative of a probability for a disorder linked to the immune system to affect the developing organism from the values of the set of parameters calculated at step a) by means of a classifier built by automatic learning from a reference data set representative of genes alleles of reference progenitors, the disorder linked to the immune system having affected only a portion of developing organisms issued from said reference progenitors.

2. The system of claim 1, wherein the gene allele is related to the immune system.

3. The system according to claims 1 or 2, wherein the disorder linked to the immune system is a disorder occurring during a pregnancy of the organism to be pregnant with the developing organism, said progenitors being selected from a couple of individuals and a couple of individuals and an egg donor.

4. The system according to any of claims 1 to 3, configured for carrying a step of acquiring the data sets prior step a).

5. The system according to any of claims 1 to 4, wherein the developing organism is selected from an embryo and a fetus.

6. The system according to any of claims 1 to 5, wherein the disorder linked to the immune system is an obstetrical complication selected from a miscarriage, a recurrent spontaneous abortion, an implantation failure and a stillbirth.

7. The system according to any of claims 1 to 6, wherein at least one of the parameters is a numerical representation of both a gene allele and of the affinities of peptides to said gene allele.

8. The system according to claim 7, wherein at least one of the parameters is a vector of a matrix calculated by decomposing an interaction matrix, each value of the interaction matrix corresponding to the affinity between a gene allele and a peptide.

9. The system according to any of claims 1 to 8, wherein at least one of the parameters of the set of parameters is a distance gene alleles at a given locus.

10. The system according to any of claims 1 to 9, wherein at least one of the parameters of the set of parameters is a norm of a vector of distances between gene alleles at a given locus, and optionally the mean of distances between gene alleles having the same locus.

11. The system according to any of claims 2 to 10, wherein the immune system related gene alleles are MHC gene alleles.

12. The system according to claim 11, wherein the immune system related gene alleles are selected at least from HLA-A alleles, HLA-B alleles, HLA-C alleles, HLA-DRB1 alleles, HLA-DPB1 alleles, HLA-DQB1 alleles, HLA-E alleles, HLA-G alleles and KIR alleles.

13. The system according to claims 11 or 12, wherein the immune system related gene alleles comprise a KIR gene allele and wherein the immune system related gene alleles are a KIR AB gene alleles and a KIR BB gene alleles, and wherein the values of the parameters representing said gene alleles are equal.

14. The system according to any of claims 1 to 13, wherein the progenitors comprise a potential progenitor selected from a set of potential progenitors, and wherein step b) is repeated for every potential progenitor of the set of potential progenitors, the processor unit being configured for further carrying a step c) of ranking the potential progenitors depending on the probability calculated at step b).

15. The system according to claim 14, wherein the potential progenitor is selected from sperm donors and egg donors.

16. A method for calculating a value representative of a probability for a disorder linked to the immune system to affect a developing organism issued from at least two progenitors, one of the at least two progenitors being an organism to be pregnant with the developing organism, comprising steps of:

a) calculating values of a set of parameters, each parameter being a numerical representation of at least a gene allele, the values of the set of parameters being calculated from at least two data sets, said data sets being representative of:

- gene alleles of the progenitors, one of the progenitors being the organism to be pregnant with the developing organism, or
- gene alleles of the organism to be pregnant and gene alleles of the developing organism,

and said data sets being stored in the memory unit,
b) calculating the value representative of a probability for a disorder linked to the immune system to affect the developing organism from the values of the set of parameters calculated at step a) by means of a classifier built by automatic learning from a reference data set representative of genes alleles of reference progenitors, the disorder linked to the immune system having affected only a portion of the developing organisms issued by said reference progenitors.

**17.** A computer program product, comprising computer instructions for carrying out a method according to claim 16.

**18.** A computer-readable storage medium having stored therein a computer program product according to claim 17.

**19.** A system for building a classifier, said system comprising a processor unit and a memory unit, the memory unit comprising a reference data set, said reference data set comprising data representative of gene alleles of a plurality of reference progenitors, a disorder linked to the immune system having affected only a portion of developing organisms issued from the reference progenitors, said processor being configured for carrying steps of:

e) calculating parameters from the reference data set, each parameter being a numerical representation of at least a gene allele,
f) selecting a subset of parameters among the parameters calculated at step e), the parameters of the selected subset being selected as being the most highly correlated to a disorder linked to the immune system,
g) training a classifier with the parameters of the selected subset.

| calculating parameters from the reference data set | ~101 |

| selecting highly correlated parameters | ~102 |

| training the classifier with the selected parameters | ~103 |

Figure 1

Figure 2

number of parameters

Figure 3

Figure 4

Figure 5

Europäisches
Patentamt

European
Patent Office

Office européen
des brevets

# EUROPEAN SEARCH REPORT

Application Number

EP 18 30 6444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/43851 A1 (NAT UNIV IRELAND CORK [IE]; BRIEN MARGARET O [IE] ET AL.) 2 September 1999 (1999-09-02) * whole document, in particular abstract, claim 1, table 1-2, 4, p. 24, 25 * | 1-19 | INV. G16B20/20 G16H50/20 |
| X | EMMERY JOHANNE ET AL: "Associations between fetal HLA-G genotype and birth weight and placental weight in a large cohort of pregnant women - Possible implications for HLA diversity", JOURNAL OF REPRODUCTIVE IMMUNOLOGY, ELSEVIER SCIENCE IRELAND LTD, IE, vol. 120, 20 February 2017 (2017-02-20), pages 8-14, XP085004150, ISSN: 0165-0378, DOI: 10.1016/J.JRI.2017.02.002 * whole document, in particular 3.3 * | 1-19 | |
| X | WEI FAN ET AL: "Relationship between HLA-G polymorphism and susceptibility to recurrent miscarriage: A meta-analysis of non-family-based studies", JOURNAL OF ASSISTED REPRODUCTION AND GENETICS., vol. 31, no. 2, 19 December 2013 (2013-12-19), pages 173-184, XP055585271, US ISSN: 1058-0468, DOI: 10.1007/s10815-013-0155-2 * the whole document * | 1-6,11, 12,16 | TECHNICAL FIELDS SEARCHED (IPC) G16B G16H |

-/--

The present search report has been drawn up for all claims

| Place of search | Date of completion of the search | Examiner |
|---|---|---|
| Munich | 6 May 2019 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 30 6444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PERSSON GRY ET AL: "HLA class Ib in pregnancy and pregnancy-related disorders", IMMUNOGENETICS, SPRINGER VERLAG, BERLIN, DE, vol. 69, no. 8, 11 July 2017 (2017-07-11), pages 581-595, XP036288180, ISSN: 0093-7711, DOI: 10.1007/S00251-017-0988-4 [retrieved on 2017-07-11] * the whole document * | 1-19 | |
| A | US 2015/186468 A1 (WEILL LAWRENCE R [US] ET AL) 2 July 2015 (2015-07-02) * whole doc, in particular p. 21-25 * | 1-19 | |
| A | COLUCCI FRANCESCO ED - TAKAHAMA YOUSUKE YOUSUKE TAKAHAMA@NIH GOV ET AL: "The role of KIR and HLA interactions in pregnancy complications", IMMUNOGENETICS, SPRINGER VERLAG, BERLIN, DE, vol. 69, no. 8, 10 July 2017 (2017-07-10), pages 557-565, XP036288312, ISSN: 0093-7711, DOI: 10.1007/S00251-017-1003-9 [retrieved on 2017-07-10] * the whole document * | 1-19 | TECHNICAL FIELDS SEARCHED (IPC) |

The present search report has been drawn up for all claims

| Place of search | Date of completion of the search | Examiner |
|---|---|---|
| Munich | 6 May 2019 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 30 6444

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2019

| Patent document cited in search report | | Publication date | Patent family member(s) | | Publication date |
|---|---|---|---|---|---|
| WO 9943851 | A1 | 02-09-1999 | AU | 2636399 A | 15-09-1999 |
| | | | AU | 2003231618 A1 | 11-09-2003 |
| | | | CA | 2321223 A1 | 02-09-1999 |
| | | | EP | 1056886 A1 | 06-12-2000 |
| | | | JP | 2003517267 A | 27-05-2003 |
| | | | WO | 9943851 A1 | 02-09-1999 |
| US 2015186468 | A1 | 02-07-2015 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARKOV, P. V. ; PYBUS, O. G.** Evolution and diversity of the human leukocyte antigen (HLA). *Evolution, medicine, and public health,* 2015, vol. 1, 1-1
- **WINTERNITZ, J. C. ; ABBATE, J. L.** Examining the evidence for major histocompatibility complex-dependent mate selection in humans and nonhuman primates. *Research and Reports in Biology,* 2015, vol. 6, 73-88
- **OBER, C.** Studies of HLA, sfertility and mate choice in a human isolate. *Human Reproduction Update,* 1999, vol. 5 (2), 103-107
- **NOWAK, I. ; WILCZYŃSKA, K. ; WILCZYŃSKI, J. R. ; MALINOWSKI, A. ; RADWAN, P. ; RADWAN, M. ; KUŚNIERCZYK, P.** KIR, LILRB and their Ligands' Genes as Potential Biomarkers in Recurrent Implantation Failure. *Archivum immunologiae et therapiae experimentalis,* 2017, vol. 65 (5), 391-399
- **HIBY, S. E. ; ASHRAFIAN-BONAB, M. ; FARRELL, L. ; SINGLE, R. M. ; BALLOUX, F. ; CARRINGTON, M. ; MOFFETT, A.** Distribution of killer cell immunoglobulin-like receptors (KIR) and their HLA-C ligands in two Iranian populations. *Immunogenetics,* 2010, vol. 62 (2), 65-73
- **LASHLEY, L. E. E. L. O. ; VAN DER WESTERLAKEN, L. A. J. ; HAASNOOT, G. W. ; DRABBELS, J. J. M. ; SPRUYT-GERRITSE, M. J. ; SCHERJON, S. A. ; CLAAS, F. H. J.** Maternal HLA-C2 and 14 bp insertion in HLA-G is associated with recurrent implantation failure after in vitro fertilization treatment. *Tissue Antigens,* 2014, vol. 84 (6), 536-544
- **PERSSON, G. ; MELSTED, W. N. ; NILSSON, L. L. ; HVIID, T. V. F.** HLA class Ib in pregnancy and pregnancy-related disorders. *Immunogenetics,* 2017, vol. 69 (8-9), 581-595
- **MEULEMAN, T. ; LASHLEY, L. E. ; DEKKERS, O. M. ; LITH, J. M. ; CLAAS, F. H. ; BLOEMENKAMP, K. W.** HLA associations and HLA sharing in recurrent miscarriage: a systematic review and meta-analysis. *Human immunology,* 2015, vol. 76 (5), 362-373
- **FAN, W. ; LI, S. ; HUANG, Z. ; CHEN, Q.** Relationship between HLA-G polymorphism and susceptibility to recurrent miscarriage: a meta-analysis of non-family-based studies. *Journal of assisted reproduction and genetics,* 2014, vol. 31 (2), 173-184
- **EMMERY, J. ; CHRISTIANSEN, O. B. ; NILSSON, L. L. ; DAHL, M. ; SKOVBO, P. ; MØLLER, A. M. ; HVIID, T. V. F.** Associations between fetal HLA-G genotype and birth weight and placental weight in a large cohort of pregnant women-Possible implications for HLA diversity. *Journal of reproductive immunology,* 2017, vol. 120, 8-14